# EUROPEAN PATENT SPECIFICATION

(11) **EP 1 857 451 B1**
(45) Date of publication and mention of the grant of the patent: **21.07.2010**
(21) Application number: 06009313.5
(22) Date of filing: 05.05.2006
(51) Int. Cl.: C07D 333/20

(54) **A process for the preparation of an intermediate useful for the asymmetric synthesis of (+)duloxetine**
Verfahren zur Herstellung eines Zwischenprodukts für die asymmetrische Synthese von (+)Duloxetine
Procédé pour la préparation d'un intermédiare pour la production asymmetirique du (+)Duloxetine

(43) Date of publication of application: 21.11.2007
(73) Proprietor: FIDIA FARMACEUTICI S.p.A., 35031 Abano Terme (PD) (IT)
(72) Inventor: Poggiali, Andrea, 20024 Garbagnate (MI) (IT); Pizzocaro, Francesco, 20024 Garbagnate (MI) (IT); Tubertini, Paolo, 20024 Garbagnate (MI) (IT)
(74) Representative: Minoja, Fabrizio

(56) References cited:
- EP-A- 0 650 965
- WO-A-2004/056795
- WO-A-2006/027998
- WO-A-2006/045255
- DEETER J ET AL: "ASYMMETRIC SYNTHESIS AND ABSOLUTE STEREOCHEMISTRY OF LY248686" 26 November 1990 (1990-11-26), TETRAHEDRON LETTERS, ELSEVIER, AMSTERDAM, NL, PAGE(S) 7101-7104 , XP001119089 ISSN: 0040-4039 Scheme * page 7102 *
- SORBERA L A ET AL: "Duloxetine Oxalate" DRUGS OF THE FUTURE, BARCELONA, ES, vol. 25, no. 9, 2000, pages 907-916, XP002350273 ISSN: 0377-8282

## Description

### Field of the invention

The present invention provides a process for the resolution of the key intermediate, N,N-dimethyl-3-(1-naphthalenyloxy)-3-(2-thienyl)propanamine, useful in the synthesis of duloxetine in enantiomerically pure form.

### Background of the invention

Duloxetie, (S)-(+)-N-methyl-3-(1-naphthalenyloxy)propanamine, having formula (I), is an useful anti-depressant.

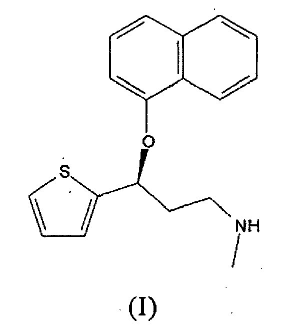

Duloxetine is described in US 5,023,269, 4,956,388, and in Tetrahedron Letters, 31, (49), 7101-04, 1990. Seven different routes of synthesis have also been reported in Drugs of the Future 2000, 25(9) 907-916. These syntheses comprise either a resolution of a key intermediate or a stereospecific reduction of a keto group to alcohol group.

WO 2004/056795 discloses that a particular chiral intermediate for the synthesis of (+)duloxetine, namely 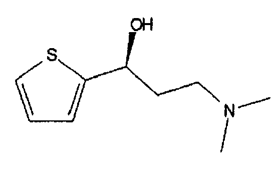 undergoes considerable racemisation during coupling with a 1-naphthyl halide under the reaction conditions used in the prior art. In particular, in the presence of a strong base, such as sodium hydride, and a protic polar solvent, such as DMSO, the anion at the alpha position of the thiophene ring can be generated , causing partial or complete racemisation of the condensed product. Early resolution was not therefore considered suitable.

However, it is well known that, to make the process economically advantageous, it is important to carry out the resolution as early as possible and not on the final compound. It is also important to minimize the risk of racemization during the subsequent steps, selecting the suitable intermediate. On the other hand, resolving agents are often expensive and there is also an advantage in limiting the amounts of these reactants.

WO 2006/045255 and WO 2006/027998 disclose a process for the preparation of (+)-duloxetine comprising the resolution of *N*-methyl duloxetine with a chiral acid, followed by demethylation.

It has now been found a process which is advantageous since it avoids racemisation and it is more economic, yielding an enantiomerically pure form of (+)duloxetine. In particular, the process of the invention achieves the above described advantages, by carrying out resolution not as a final step in the reaction process, but resolving the intermediate N,N-dimethyl-3-(1-naphthalenyloxy)-3-(2-thienyl)propanamine (N-methylduloxetine), in addition with a less than stoichiometric amount of the expensive resolving agent.

### Description of the invention

The present invention, therefore, provides a process for the preparation of (+)duloxetine, or an acid addition salt thereof, comprising:
(i) Resolving racemic (±)N-methyl duloxetine with a less than stoichiometric amount of a chiral acid in combination with suitable amounts of a hydrohalogen acid to give a salt of the chiral acid and (+)N-methyl duloxetine, substantially free from (-)N-methyl duloxetine;
(ii) Demethylating the (+)N-methyl duloxetine prepared in (i) to obtain (+)duloxetine or another acid addition salt in enantiomerically pure form.

The resolution step (i) is achieved with a combination of a suitable chiral acid and a hydrohalogen acid, in a suitable molar ratio with (±)N-methyl duloxetine, in a suitable solvent. The chiral acid can typically be selected from the group consisting of mandelic acid, tartaric acid, di-p-toluyl tartaric acid, dibenzoyl tartaric acid, camphorsulfonic acid and the like. Other suitable chiral acids may be selected by those skilled in the art and the use thereof in a process as described above falls within the scope of the present invention. hydrohalogen acids include hydrochloric, hydrobromic and hydroiodic acids, and preferably hydrochloric and hydrobromic acids. Preferably the chiral acid used in the process according to the invention is (D)-(-)-tartaric acid. Suitably, the molar ratio (±)N-methyl duloxetine: chiral acid: hydrohalogen acid ranges between 1: 0.5: 0.5 and 1: 1: 1. Preferably the molar ratio (±)N-methyl duloxetine: chiral acid: hydtohalogen acid used in the process of the invention is 1.00: 0.70: 0.43. Suitably, the solvent is a lower alkanol, such as methanol or ethanol, although other suitable solvents can be used as well. A preferred solvent is ethanol.

Said salts of a chiral acid and (+)N-methyl duloxetine, substantially free from (-)N-methyl duloxetine, are useful intermediates for the preparation of the free base or another acid addition salt of (+)duloxetine.

Suitable salts provided by the invention include (+)N-methyl duloxetine mandelate, (+)N-methyl duloxetine tartrate, (+)N-methyl duloxetine di-p-toluyl tartrate, (+)N-methyl duloxetine dibenzoyl tartrate, (+)N-methyl duloxetine camphor sulfonate and the like. A preferred salt of the invention is (+)N-methyl duloxetine tartrate.

Intermediate salts prepared according to the invention can be converted to (+)duloxetine free base or another acid addition salt according to step (ii) of said process. Suitably, an intermediate salt of the chiral acid and (+)N-methyl duloxetine are treated with a base, such as sodium hydroxide, to yield the free base, and then demethylated to obtain (+)-duloxetine in enantiomerically pure form. The free base itself can, if desired, be converted into an acid addition salt thereof. Suitable acid addition salts are obtained by reaction either with pharmaceutically acceptable organic acids or with inorganic acids. Examples of inorganic acids include hydrochloric, hydrobromic, hydroiodic, sulfuric and phosphoric acid. Examples of organic acids include para-toluenesulfonic, methanesulfonic, oxalic, para-bromophenylsulfonic, carbonic, succinic, citric, benzoic and acetic acid. Said pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phtbalate, terephthalate, sulfonate, xylenesulfonate, phenylacetate, phenylpropionate, phenylbutyrate, citrate, lactate, -hydroxybutyrate, glycollate, maleate, tartrate, methanesulfonate, propanesulfonates, naphthalene-1-sulfonate, naphthalene-2-sulfonate, mandelate and similar salts. Preferred salts are the hydrochloride, hydrobromide, oxalate and mandelate. The hydrochloride is particularly preferred.

A particularly preferred process according to the present invention comprises:
(i) resolving racemic (±)N-methyl duloxetine with a combination of (D)-(-)-tartaric acid and hydrochloric acid to give (+)N-methyl duloxetine tartrate, substantially free from (-)N-methyl duloxetine; and
(ii) converting (+)N-methyl duloxetine tartrate prepared in step (i) to (+)duloxetine hydrochloride.

The process of the invention preferably yields (+)N-methyl duloxetine in substantially pure enantiomeric form. Thus the ratio of (+)N-methyl duloxetine: (-)N-methyl duloxetine as prepared by the present invention may be at least about 95:5, preferably at least about 98:2, more preferably at least about 99:1. Preferably (+)duloxetine prepared by the process of the invention has an enantiomeric purity of at least about 99%, more particularly at least about 99.5%.

The yield in (+)N-methyl duloxetine achieved by the process of the invention can be increased by carrying out one or more, for example two or three, re-crystallisations in step (i). To this end, a crystalline salt obtained by resolution may be dissolved in a solvent f and the salt is recrystallised from the resulting solution. In this way the proportion of a salt with (+)N-methyl duloxetine can be increased until it is substantially pure, i.e. when only the salt with (+)N-methyl duloxetine is present.

According to the invention (+)duloxetine of formula (I): 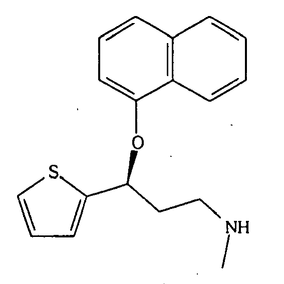 is prepared by a process comprising:
i) reacting a compound of formula (II), 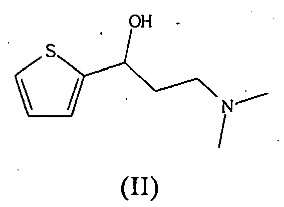 with a compound of formula (III) 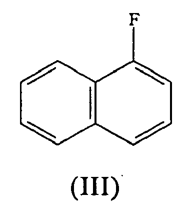 in a suitable solvent, to yield the compound of formula (IV)

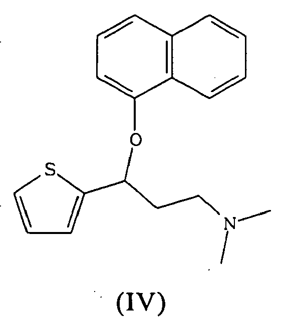

The compound of formula (II) can be prepared by known methods. Preferably, standard Mannich reaction conditions are employed to synthesize the corresponding Mannich base from the appropriate ketone, formaldehyde and dimethylamine, which is then reduced with a hydride reducing agent, such as sodium borohydride, under standard conditions.

The compound (IV) is preferably synthesized by treating compound (II) with an alkali metal hydride to form the corresponding alkali metal salt, which is then reacted with compound (III).

This reaction is carried out by combining approximately equimolar quantities to a slight excess of the alkali metal hydride with the compound (II) to provide the corresponding alkali metal salt. Typical alkali metal hydrides include sodium hydride and potassium hydride. The compound is then reacted with an equimolar quantity to slight excess of the compound (III). The reaction is substantially complete in a period ranging from 10 minutes to about 24 hours when carried out at a temperature in the range of about 25°C to about 150°C. The reaction is usually complete within about 30 minutes to about 6 hours at a temperature ranging from about 75°C to about 125°C. The most preferred temperatures range is included between 85°C and 95°C and the reaction time is preferably from 1 hour to 2 hours. Examples of suitable solvents include DMA, DMPU or the like. The product may be isolated by conventional procedures. Typically, the mixture is diluted with water, hydrochloric acid is added and the aqueous phase extracted with a water immiscible organic solvent such as hexane, diethyl ether, ethyl acetate and the like. Sodium hydroxide is added to the water solution, and the alkaline solution is extracted with a water immiscible organic solvent such as cyclohexane, diethyl ether, ethyl acetate and the like. The organic extracts are combined, washed with water and concentrated under vacuum. The remaining oil (oxalate salt) is isolated.

The resolution of compound (IV) is carried out obtained as described above, with a less than stoichiometric amount of a suitable chiral acid in combination with suitable amounts of hydrohalogen acids in a suitable solvent, to give compound (V) in enantiomerically pure form.

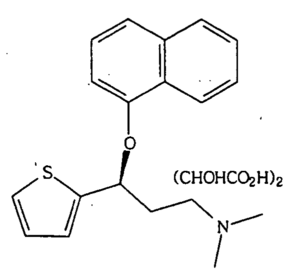

The most preferred molar ratio compound (IV): chiral acid:
hydrohalogen acid is 1: 0.70: 0.43.

The conversion of the compound (V) to (+)duloxetine (I) by demethylation, is carried out in the presence of a reagent such as phenyl chloroformate or trichloroethyl chloroformate and the like, to provide the corresponding intermediate, which is then hydrolyzed to afford (+)duloxetine, or an acid addition salt thereof, according to known methods. Preferably (+)duloxetine is isolated as the oxalate.

Finally, (+)duloxetine oxalate is converted to the hydrochloride by known methods.

The following examples illustrate the method of the invention.

### Example 1

### 3-Dimetbylammo-1-(2-thienyl)-1-propanone hydrochloride

A mixture of 2-acetylthiophene (100 g, 0.79 mol), dimethylamine hydrochloride (84.3 g, 1.03 mol), Para formaldehyde (31.4 g, 0.35 mol) and 35% w/w hydrochloric acid (2 g, 0.02 mol) in ethanol (130 ml) was refluxed for 8 hours. The mixture was then cooled to about 20-25°C and diluted with ethanol (160 ml) and acetone (745 ml). The mixture was stirred for 2 hours and the solid was collected by filtration to yield 140 g (80%) of 3-dimethylamino-1-(2-thienyl)-1-propanone hydrochloride as a colorless crystalline solid.

### Example 2

### N,N-dimethyl-3-hydroxy-3-(2-thienyl)propanamine

30% w/w NaOH (73 g, 0.55 mol) was added to a mixture of 3-dimethylamino-1-(2-thienyl)-1-propanone hydrochloride (100 g, 0.46 mol) in water (150 ml), at room temperature, and the free base was extracted with toluene (170 ml). A mixture of 2-propanol (170 ml) and water (23 ml) was added to the resulting solution, then a solution of sodium borohydride (17 g, 0.45 mol) in water (58 ml) was added drop wise, maintaining the temperature between 20°C and 35°C. After addition was completed, the mixture was stirred for eight hours at 20-35°C. Acetone (100 ml) to was added the solution and then the mixture of 2-propanol and acetone was removed in vacuo. The remaining solution was diluted with water (75 ml) and toluene (200 ml). The organic phase was washed with water and then partially concentrated in vacuum. The mixture was cooled to about 0-5°C and then was stirred for one hour and the solid was collected by filtration to yield 67 g (80%) of N,N-dimethyl-3-hydroxy-3-(2-thienyl)propanamine as a colorless crystalline solid.

### Example 3

### N,N-dimethyl-3-(1-naphthalenyloxy)-3-(2-threnyl)propanamine oxalate

A solution of N,N-dimethyl-3-hydroxy-3-(2-thienyl)propanamine in dimethylacetamide (140 ml) and dimethylpropyleneurea was added (60 ml) to a mixture of 60% NaH (25 g, 0.63 mol) in N,N-dimethylpropylenurea (80 ml) at 0-25°C. After addition was completed, the mixture was slowly heated at 85-95°C; 1-fluoronaphthalene (91 g, 0.62 mol) was added dropwise to the mixture and the resulting solution was stirred for 2 hours at 85-95°C. The mixture was then cooled to about 20-25°C and added to cool water maintaining the temperature between 0°C and 15°C. After completion of the addition, the solution was stirred for 1 hour. 35% w/w HCl (141 g, 1.35 mol) was added dropwise to the solution. The solution was extracted twice with hexane. 30% w/w NaOH (198 g, 1.49 mol) was added dropwise to the water solution. The solution was extracted twice with cyclohexane. The organic phase was washed twice with water and then concentrated under vacuum. To the remaining oil in methanol (200 ml) was added, at reflux temperature, a solution of oxalic acid dihydrate (69 g, 0.55 mol) in methanol (250 ml). After completion of the addition, the solution was cooled to about 0-5°C and then was stirred for one hour and the solid was collected by filtration to yield 163 g (75%) of N,N-dimethyl-3-(1-naphthalenyloxy)-3-(2-thienyl)propanamine oxalate as a colorless crystalline solid.

### Example 4

### (S)-(+)-N,N-dimethyl-3-(1-naphthalenyloxy)-3-(2-thienyl)propanamine tartrate.

50% w/w Potassium hydroxide (59 g, 0.53 mol) was added, at 30-40°C, to a mixture of N,N-dimethyl-3-(1-naphthalenyloxy)-3-(2-thienyl)propanamine oxalate (100 g, 0.25 mol) in water (200 ml) and the free base was extracted twice with hexane (150 ml). The extracts were combined, washed twice with water and then concentrated in vacuum, (D)-(-)-tartaric acid (26 g, 0.17 mol) t was added to the remaining oil in ethanol (370 ml), at room temperature. The mixture was heated at 50-55°C and stirred until complete solution. 35% w/w Hydrochloric acid (11 g, 0.11 mol) was added to the solution. After completion of the addition, the solution was cooled to about 15-25°C, stirred for 8 hours and the solid was collected by filtration to yield 35 g (30%) of (S)-(+)-N,N-dimethyl-3-(1-naphthalenyloxy)-3-(2-thienyl)propanamine tartrate as a colorless crystalline solid (S:R ratio 95:5). Recrystallization of the crystals from ethanol gave a product with a ratio S:R higher than 99:1.

### Example 5

### (S)-(+)-N-methyl-3-(1-naphthalenyloxy)-3-(2-thienyl)propanamine oxalate

50% w/w Potassium hydroxide (51 g, 0.46 mol) was added, at 30-40°C, to a mixture of (S)-(+)-N,N-dimethyl-3-(1-naphthalenyloxy)-3-(2-thienyl)propanamine tartrate (100 g, 0.22 mol) in water (250 ml), and the free base was extracted twice with toluene (250 ml). The organic phase was washed twice with water (30 ml), concentrated to half volume under vacuum and heated to 50-55°C. Then diisopropylethylamine (28 g, 0,22 mol) was added, followed by the dropwise addition of phenyl chloroformate (42.4 g, 0.27 mol). The mixture was stirred at 50-55°C for 4 hours, and then cooled at room temperature. Sodium bicarbonate solution 1% (100 ml) was added, the mixture was stirred for ten minutes at 20-25°C and the phases were separated. The organic phase was washed with 1% hydrochloric acid (50 ml), and then washed with sodium bicarbonate solution 1% (100 ml). The washed organic phase was evaporated under vacuum. 30% w/w Sodium hydroxide (87 g, 0.65 mol) was added to the remaining oil in dimethylsulfoxide (500 ml), at 50-60°C, and the mixture was stirred for 6 hours at 50-60°C. The mixture was added dropwise to cool water (400 m1), and acidified to pH < 3 by addition of 35% w/w HCI. The solution was extracted twice with hexane and the aqueous phase was alkalinized to pH >12 by addition of 30% w/w NaOH. The solution was extracted twice with cyclohexane. The organic phase was washed twice with water and then concentrated in vacuum. A solution of oxalic acid dihydrate (27 g, 0.21 mol) in methanol (150 ml) was added, under reflux temperature to the remaining oil in methanol (400 ml) . After addition was completed, the solution was cooled to about 0-5°C and then was stirred for 1 hour and the solid was collected by filtration to yield 46 g (55%) of (S)-(+)-N-methyl-3-(l-naphthalenyloxy)-3-(2-thie-nyl)propanamine oxalate as a colorless crystalline solid.

### Example 6

### (S)-(+)-N-methyl-3-(1-naphthalenyloxy)-3-(2-thienyl)propanamine hydrochloride

50% w/w KOH (61.2 g, 0.55 mol) was added, at 30-40°C, to a mixture of (S)-(+)-N-methyl-3-(1-naphthalenyloxy)-3-(2-thienyl)propanamine oxalate (100 g, 0.26 mol) in water (200 ml) and the free base was extracted twice with cyclohexane (500 ml). The extracts were combined, washed twice with water and then concentrated under vacuum. 35% w/w Hydrochloric acid (27.1 g, 0.26 mol) was added, at room temperature, to the remaining oil in ethyl acetate (295 ml). After addition was completed, the solution was cooled to about 0-5°C and then was stirred for 2 hours and the solid was collected by filtration to yield 43 g (50%) of (S)-(+)-N-methyl-3-(1-naphthalenyloxy)-3-(2-thienyl)propanamine hydrochloride as a colorless crystalline solid (S:R ratio higher than 99.5:0.5).

## Claims

1. A process for the preparation of (+)duloxetine, or an acid addition salt thereof, comprising:
i) reacting a compound of formula (II), 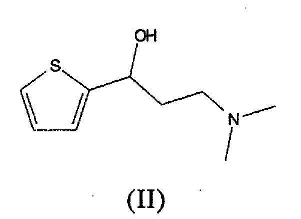 with a compound of formula (III) 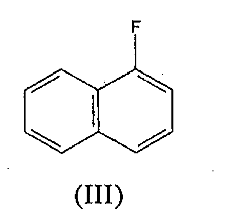 in a suitable solvent, to yield racemic (±)N-methyl duloxetine of formula (IV) 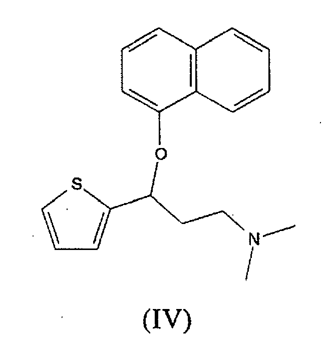
(ii) resolving racemic (±)N-methyl duloxetine of **formula IV** with a less than stoichiometric amount of a chiral acid in combination with suitable amounts of a hydrohalogen acid in a suitable solvent to give a salt of the chiral acid and (+)N-methyl duloxetine, substantially free from (-)N-methyl duloxetine;
(iii) Demethylating the (+)N-methyl duloxetine prepared in step (ii) to give (+)duloxetine or another acid addition salt in enantiomerically pure form.

2. A process according to claim 1, wherein the chiral acid is selected from mandelic acid, tartaric acid, di-p-toluyl tartaric acid, dibenzoyl tartaric acid and camphor sulfonic acid.

3. A process according to claim 2, wherein the chiral acid is tartaric acid.

4. A process according to claim 1, wherein the hydrohalogen acid is selected from hydrochloric acid, hydrobromic acid, and hydroiodic acid.

5. A process according to claim 4, wherein the hydrohalogen acid is hydrochloric acid.

6. A process according to claim 1, wherein the molar ratio (±)N-methyl duloxetine: chiral acid: hydrohalogen acid ranges between 1: 0.5: 0.5 and 1: 1: 1.

7. A process according to claim 6, wherein the molar ratio (±)N-methyl duloxetine: chiral acid: hydrohalogen acid is 1: 0.70: 0.43.

8. A process according to any one of claims 1 to 7, wherein the suitable solvent for step (ii) is methanol or ethanol.

9. A process according to claim 8, wherein the solvent is ethanol.

## Patentansprüche

1. Verfahren zur Herstellung von (+)Duloxetin oder eines Säureadditionssalzes davon, umfassend:
(i) zur Reaktion bringen einer Verbindung der Formel (II), 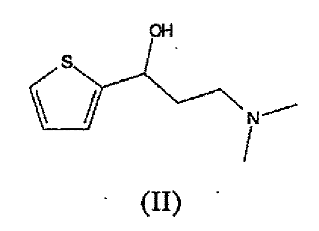 mit einer Verbindung der Formel (III) 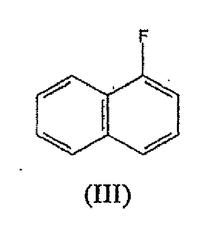 in einem geeigneten Lösungsmittel, um racemisches (±)N-Methyl-Duloxetin der Formel (IV) hervorzubringen 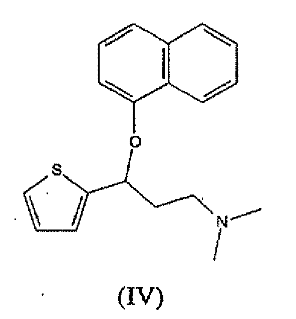
(ii) Trennen des racemischen (±)N-Methyl-Duloxetins der Formel IV mit einer weniger als stöchiometrischen Menge einer chiralen Säure in Kombination mit geeigneten Mengen einer Halogenwasserstoffsäure in einem geeigneten Lösungsmittel, um ein Salz der chiralen Säure und (+)N-Methyl-Duloxetin, im Wesentlichen frei von (-)N-Methyl-Duloxetin, zu ergeben;
(iii) Demethylieren des in Schritt (ii) hergestellten (+)N-Methyl-Duloxetins, um (+)Duloxetin oder ein anderes Säureadditionssalz in enantiomerenreiner Form zu ergeben.

2. Verfahren nach Anspruch 1, wobei die chirale Säure ausgewählt ist aus Mandelsäure, Weinsäure, Di-p-Toluyl-Weinsäure, Dibenzoyl-Weinsäure und Camphersulfonsäure.

3. Verfahren nach Anspruch 2, wobei die chirale Säure Weinsäure ist.

4. Verfahren nach Anspruch 1, wobei die Halogenwasserstoffsäure ausgewählt ist aus Chlorwasserstoffsäure, Bromwasserstoffsäure und Jodwasserstoffsäure.

5. Verfahren nach Anspruch 4, wobei die Halogenwasserstoffsäure Chlorwasserstoffsäure ist.

6. Verfahren nach Anspruch 1, wobei das molare Verhältnis von (±)N-Methyl-Duloxetin : chiraler Säure : Halogenwasserstoffsäure zwischen 1 : 0,5 : 0,5 und 1:1:1 liegt.

7. Verfahren nach Anspruch 6, wobei das molare Verhältnis von (±)N-Methyl-Duloxetin : chiraler Säure : Halogenwasserstoffsäure 1 : 0,70 : 0,43 beträgt.

8. Verfahren nach einem der Ansprüche 1 bis 7, wobei das geeignete Lösungsmittel für Schritt (ii) Methanol oder Ethanol ist.

9. Verfahren nach Anspruch 8, wobei das Lösungsmittel Ethanol ist.

## Revendications

1. Procédé de préparation de (+)duloxétine, ou d'un sel d'addition acide de celle-ci, comprenant :
i) la réaction d'un composé de formule (II) 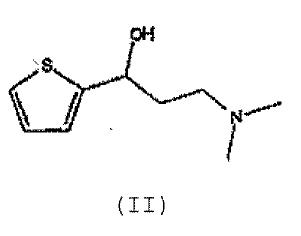 avec un composé de formule (III) 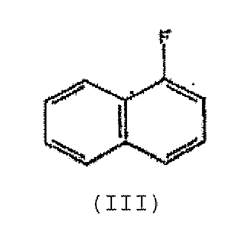 dans un solvant approprié, pour donner la (±)N-méthyl duloxétine racémique de formule (IV) 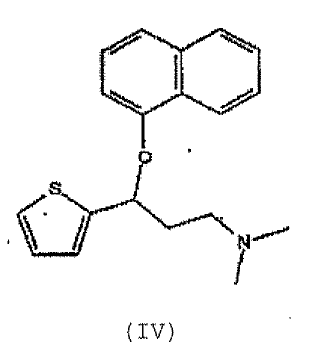
(ii) la résolution de la (±)N-méthyl duloxétine racémique de formule (IV) avec une quantité moins que stoechiométrique d'un acide chiral en combinaison avec des quantités appropriées d'un acide halohydrique dans un solvant approprié pour donner un sel de l'acide chiral et la (+)N-méthyl duloxétine, substantiellement exempte de (-)N-méthyl duloxétine ;
(iii) la déméthylation de la (+)N-méthyl duloxétine préparée à l'étape (ii) pour donner la (+)duloxétine ou un autre sel d'addition acide sous une forme énantiomériquement pure.

2. Procédé selon la revendication 1, dans lequel l'acide chiral est choisi parmi l'acide mandélique, l'acide tartrique, l'acide di-p-toluyltartrique, l'acide dibenzoyltartrique et l'acide camphorsulfonique.

3. Procédé selon la revendication 2, dans lequel l'acide chiral est l'acide tartrique.

4. Procédé selon la revendication 1, dans lequel l'acide halohydrique est choisi parmi l'acide chlorhydrique, l'acide bromhydrique, et l'acide iodhydrique.

5. Procédé selon la revendication 4, dans lequel l'acide halohydrique est l'acide chlorhydrique.

6. Procédé selon la revendication 1, dans lequel le rapport molaire (±)N-méthyl duloxétine:acide chiral:acide halohydrique est dans la plage entre 1:0,5:0,5 et 1:1:1.

7. Procédé selon la revendication 6, dans lequel le rapport molaire (±)N-méthyl duloxétine:acide chiral:acide halohydrique est 1:0,70:0,43.

8. Procédé selon l'une quelconque des revendications 1 à 7, dans lequel le solvant approprié pour l'étape (ii) est le méthanol ou l'éthanol.

9. Procédé selon la revendication 8, dans lequel le solvant est l'éthanol.
